# EUROPEAN PATENT SPECIFICATION

(11) **EP 3 985 009 B1**
(45) Date of publication and mention of the grant of the patent: **08.05.2024**
(21) Application number: 20808820.3
(22) Date of filing: 25.03.2020
(51) Int. Cl.: C07D 495/04, A61P 7/02, A61K 31/4365, A61P 9/00, A61P 9/04, A61P 9/10

(54) **B CRYSTAL FORM OF TETRAHYDROTHIENOPYRIDINE COMPOUND, PREPARATION METHOD THEREFOR, COMPOSITION AND APPLICATION**
B-KRISTALLFORM EINER TETRAHYDROTHIENOPYRIDINVERBINDUNG, VERFAHREN ZU IHRER HERSTELLUNG, ZUSAMMENSETZUNG UND ANWENDUNG
FORME CRISTALLINE B DE COMPOSÉ DE TÉTRAHYDROTHIÉNOPYRIDINE, SON PROCÉDÉ DE PRÉPARATION, COMPOSITION ET APPLICATION

(30) Priority: 17.05.2019 CN 201910412233
(43) Date of publication of application: 20.04.2022
(73) Proprietor: Chengdu Shibeikang Biomedical Technology Co., Ltd., Chengdu, Sichuan 611731 (CN)
(72) Inventor: CEN, Guodong, Chengdu, Sichuan 611731 (CN); YANG, Maoting, Chengdu, Sichuan 611731 (CN); TAN, Shaojun, Chengdu, Sichuan 611731 (CN)
(74) Representative: Ipside
(86) International application number: PCT/CN2020/081091
(87) International publication number: WO 2020/233226

## Description

### Technical Field

The present invention relates to medical field, particularly a crystal form B of a tetrahydrothienopyridine compound (S)-2-(2-chlorophenyl)-2-((S)-2-oxo-2,6,7,7a-tetrahydrothiophene[3,2-c]pyridine-5(4H)yl)methyl acetate, and a preparation method , composition and application thereof.

### Background of the Invention

According to data from Report on Cardiovascular Diseases in China 2017, prevalence and mortality of cardiovascular diseases (CVD) in China have been on the rise in recent years. It is estimated that there are 290 million people with CVDs, including 13 million people with stroke, 11 million people with coronary heart disease, 4.5 million people with heart failure and 270 million people with hypertension. According to the latest JAMA survey data published in JAMA Cardiology, the total number of deaths due to CVDs in China in 2016 was as high as 3.97 million, accounting for more than 40% of the total number of deaths due to disease among the population. CVDs have become the dominant factor of death due to disease in China, higher than the number of deaths due to tumors. Among them, cardiovascular and cerebrovascular thrombotic diseases such as ischemic stroke (IS) and hemorrhagic stroke (HS) are the most dominant and highly fatal diseases. Meanwhile, according to the results of a recently completed cross-sectional study on stroke disease burden in China led by Ness-China, crude incidence of stroke has reached an alarming level of 345.1 cases per 100,000 people per year in China by 2013, indicating that China has become a country with high incidence of thrombotic diseases. Therefore, prevention, control and treatment of cardiovascular and cerebrovascular thrombotic diseases are particularly important under such a severe situation.

Key physiological pathways of thrombosis are platelet adhesion and aggregation. Therefore, platelet aggregation inhibitors play an important role in the treatment of thrombotic diseases, and are also the focus of research. As one of the most representative anticoagulant drugs, clopidogrel (Plavix) from Sanofi S.A. is widely used in current clinical first-line treatment and prevention of thrombotic diseases owing to good safety and rapid inhibition of platelet aggregation. Unfortunately, significant individual differences exist in efficacy of clopidogrel, especially in Asians, namely clopidogrel resistance (CPGR), which limits the application thereof. Farid et al. conducted a detailed and comprehensive study on the metabolism of clopidogrel in vivo in 2010 (J. Clin. Pharmacol 2010; 50:126-142), which revealed that the cause of CPGR is difference in CYP enzyme activity in individual liver. Specifically, clopidogrel cannot be metabolized normally in the liver of some patients, and cannot produce metabolites and optical isomers having a structure of Compound I, thus blocking subsequent further metabolism of clopidogrel into active constituents and disabling anti-coagulating action. Compound I has a chemical name of (S)-2-(2-chlorophenyl)-2-((S)-2-oxo-2,6,7,7a-tetrahydrothiophene[3,2-c]pyridine-5(4H)yl)methyl acetate.

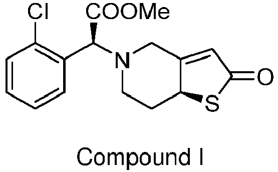

According to the metabolic pathway, pharmaceutical researchers have carried out a series of structural optimization for clopidogrel in the hope of overcoming the CPGR. Unfortunately, many bold attempts including Vicagrel and Prasugrel have failed to achieve satisfactory results due to side effects resulting from structural modification. On the other hand, Indian IPCA Laboratories heuristically and simply disclosed a preparation method for Compound I and a preliminary study on druggability thereof by patent CN104245707. As the compound is a normal CYP enzyme metabolite of clopidogrel, foreseeably the compound can overcome the CPGR and minimize other uncontrollable risks due to structural changes while inheriting traditional efficacy of clopidogrel. However, unfortunately, the patent does not deeply study a crystal form of the compound, and only selectively precipitates a crystal form of Compound I through a racemic mixture. The compound has a higher chiral purity than diastereoisomer, but has many other impurities, low content, poor crystal form stability and no druggability.

It is well known to those skilled in the art that a crystal structure of an active pharmaceutical ingredient often affects chemical stability of a drug, and different crystallization conditions and storage conditions may lead to change in the crystal structure of the compound, sometimes accompanied by generation of other crystal forms, thus affecting stability, water solubility and storage of a pharmaceutical preparation, and further affecting safety and reliability of the drug. Therefore, it is necessary to go into the crystal form of Compound I. It is urgent for those skilled in the art to provide a crystal form of Compound I with good stability, high safety and good prospect of druggability as well as suitable for industrial production.

### Summary of the Invention

One of the objects of the present invention is to provide a crystal form B of (S)-2-(2-chlorophenyl)-2-((S)-2-oxo-2,6,7,7a-tetrahydrothiophene[3,2-c]pyridine-5(4H)yl)methyl acetate having a structure of Compound I (a tetrahydrothienopyridine compound) to solve problems of many impurities, low content, poor crystal form stability and no druggability in the prior art.

Another object of the present invention is to provide a preparation method for the crystal form B.

A third object of the present invention is to provide a pharmaceutical composition comprising the crystal form B.

A fourth object of the present invention is to provide an application of the crystal form B.

In order to achieve the above objects, the technical solution of the present invention is as follows:
A crystal form B of Compound I uses Cu-Kα radiation, and X-ray powder diffraction expressed at a 2θ angle has characteristic peaks at 11.21±0.2°, 12.61±0.2°, 14.69±0.2°, 16.14±0.2°, 17.81 ±0.2°, 20.22±0.2°, and 22.10±0.2°.

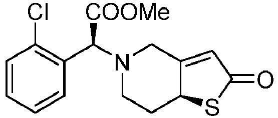

### Compound I

In one embodiment, the crystal form B uses Cu-Kα radiation, and X-ray powder diffraction expressed at the 2θ angle has characteristic peaks at 11.21±0.2°, 12.61±0.2°, 14.69±0.2°, 16.14±0.2°, 17.81±0.2°, 20.22±0.2°, 22.10±0.2°, 23.24±0.2°, 27.68±0.2°, and 28.57±0.2°.

In one embodiment, the crystal form B uses Cu-Kα radiation, and X-ray powder diffraction expressed at the 2θ angle has characteristic peaks at 11.21±0.2°, 12.61±0.2°, 14.69±0.2°, 16.14±0.2°, 17.81±0.2°, 19.42±0.2°, 20.22±0.2°, 20.76±0.2°, 22.10±0.2°, 23.24±0.2°, 24.31±0.2°, 27.01±0.2°, 27.68±0.2°, 28.57±0.2°, and 31.09±0.2°.

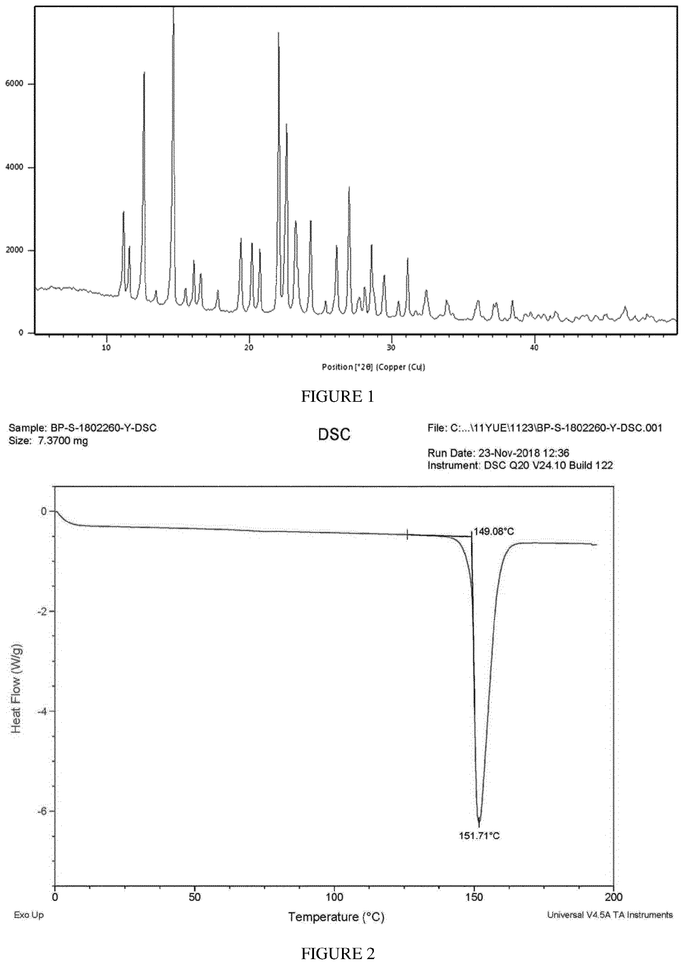

In one embodiment, the crystal form B can be characterized by an X-ray powder diffraction pattern as crystal form B shown in FIGURE 1.

In one embodiment, the crystal form B is characterized by a differential scanning calorimetry spectrogram crystal form B having an endothermic peak at 151.71±5°C.

In one embodiment, the differential scanning calorimetry spectrogram of the crystal form B is shown in FIGURE 2.

According to thermogravimetric analysis (TGA), the crystal form B of the present invention has no obvious weight loss before 100°C and at 100-170°C, indicating that the crystal form does not contain any volatile solvent, adsorbed water and water of crystallization.

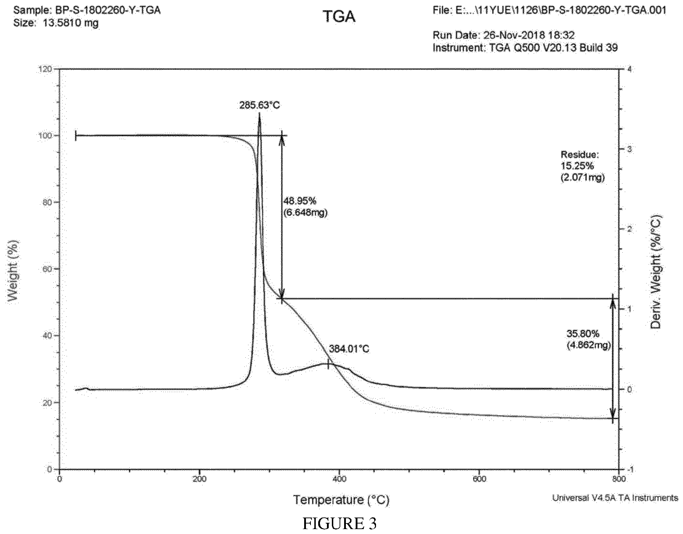

In one embodiment, a thermogravimetric analysis spectrogram of the crystal form B is shown in FIGURE 3.

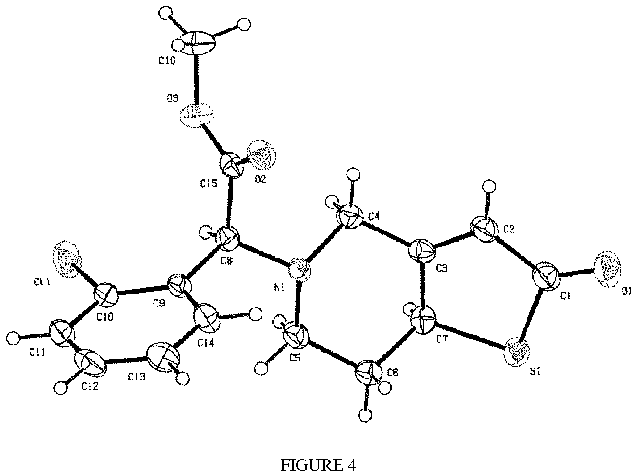

A single crystal structure of Compound I is provided in a preferred embodiment of the present invention. According to X-ray single crystal diffraction data, a crystal system of the crystal is monoclinic, a space group thereof is P21 group, and lattice constants are a=6.7206(5) Å, b=15.2446(9) Å, c=8.0941(5) Å, α=90°, β=102.618(6)°, and γ=90°. The single crystal of the crystal form B has an X-ray single crystal diffraction pattern as shown in FIGURE 4.

A method for preparing the crystal form B of Compound I comprises dissolving a crude product of any crystal form or amorphous compound of Compound I in a solvent in any way, then cooling or concentrating to crystallize, filtering, washing and drying a resulting product to obtain the crystal form.

The crude product of Compound I means that mass content of Compound I is less than 98%.

The solvent is selected from any one or more of alcohols, ketones, nitriles, ethers, esters and sulfones with carbon atom number less than or equal to 7, or a mixture of any one or more therefrom and water.

Preferably, the alcohols comprise methanol, ethanol, isopropanol, 1-propanol, 2-propanol, 1-butanol, 2-butanol and pentanol. The ketones comprise acetone, methyl ethyl ketone and methyl isopropyl ketone. The nitriles comprise acetonitrile. The ethers comprise diethyl ether, isopropyl ether, methyl tertiary butyl ether and anisole. The esters comprise methyltetrahydrofuran, ethyl formate, ethyl acetate, propyl acetate, butyl acetate, isopropyl acetate and isobutyl acetate. The sulfones comprise dimethyl sulfoxide.

Cooling crystallization is standing crystallization at -20°C to room temperature.

Concentration crystallization is concentration of the solvent by a rotary evaporator at an external temperature of 35°C for crystallization.

In some specific embodiments, a filter cake is washed with a corresponding crystallization solvent of ice, preferably a 0°C corresponding crystallization solvent.

The drying is air drying at 35°C and atmospheric pressure.

The present invention relates to a pharmaceutical composition comprising the crystal form B of Compound I and one or more pharmaceutically-acceptable carriers. The "pharmaceutically-acceptable carrier" refers to a diluent, adjuvant, excipient or vehicle administered together with a therapeutic agent, and is suitable for contact with human and/or other animal tissues within a scope of reasonable medical judgment without excessive toxicity, irritation, anaphylactic response or other problems or complications corresponding to a reasonable benefit/risk ratio.

The present invention relates to use of the crystal form B of Compound I in preparation of drugs for prevention or treatment of cardiac, cerebral and other arterial circulation disorders due to platelet hyperaggregation. The other arterial circulation disorders comprise stroke, acute coronary syndrome, atherosclerosis, myocardial infarction and confirmed peripheral arterial diseases.

Compared with the prior art, the present invention has surprisingly the following beneficial effects:
The present invention creatively finds that the crystal form B with less impurities, good stability and crystallinity and excellent reproducibility and operability can be obtained by refining crystallization of a crude single isomer of Compound I. Moreover, the crystal form B has simple preparation, controllable, cheap and easily available solvent, mild crystallization conditions and stable crystal form, and is suitable for industrial production.

In addition, the crystal form B also has unexpected technical effects: the crystal form B has stronger resistance against ADP-induced platelet aggregation, is significantly different from the crystal forms reported in the prior art, and is an advantageous crystal form with further medicinal potential. Besides, the crystal form B also has unexpected better fluidity, which enable the crystal form B to have better tabletting performance and is more conducive to control of tablet weight variation of finished products, thus further ensuring effectiveness and safety of medication.

### Brief description of the Drawings

FIGURE 1 shows an observed XRPD pattern of the crystal form B of Compound I.
FIGURE 2 shows a DSC spectrogram of the crystal form B of Compound I.
FIGURE 3 shows a TGA spectrogram of the crystal form B of Compound I.
FIGURE 4 shows a single crystal diffraction pattern of the crystal form B of Compound I.

### Detailed Description of Embodiments

The technical solution of the present invention is described below with specific embodiments. However, the protection scope of the present invention is not limited to the embodiments described herein. All reagents used are commercially available products.

In the embodiments of the present invention, according to Method 2 of General Chapter 0451 X-ray Diffraction Method in Chinese Pharmacopoeia (2015 Edition) Volume IV, X-ray powder diffraction spectra of samples are measured with Empyrean X-ray powder diffractometer of PANalytical B.V. under the following test conditions: monochromatic Cu-Kα rays (λ=1.5418 Å), scanning mode: 0/20, scanning range: 0-50°, tube voltage: 35kV, tube current: 30mA, and scanning speed: 8°/min.

In the embodiments of the present invention, according to General Chapter 0661 Thermal Analysis in Chinese Pharmacopoeia (2015 Edition) Volume IV, DSC spectrogram of the samples are measured with TA Instruments Q20 Differential Scanning Calorimeter under the following test conditions: purge gas: nitrogen, initial temperature: 0°C, final temperature: 200°C, and heating rate: 20°C/min.

In the embodiments of the present invention, according to General Chapter 0661 Thermal Analysis in Chinese Pharmacopoeia (2015 Edition) Volume IV, TGA spectrogram of the samples are measured with TA Instruments TGA Q500 Thermogravimetric Analyzer under the following test conditions: purge gas: nitrogen, initial temperature: 0°C, final temperature: 800°C, and heating rate: 20°C/min.

In the embodiments of the present invention, according to Method 1 of General Chapter 0451 X-ray Diffraction Method in Chinese Pharmacopoeia (2015 Edition) Volume IV, X-ray single crystal diffraction spectra of the samples are measured with Empyrean X-ray powder diffractometer of PANalytical B.V. under the following test conditions: monochromatic Cu-Kα rays (λ=1.5418 Å), scanning mode: 0/20, scanning range: 0-50°, tube voltage: 35Kv, and tube current: 30mA.

Those skilled in the art should understand that, for various crystal form characterization data of the present invention, due to influence of various factors such as test equipment and conditions, peak positions or relative intensities of the measured X-ray powder diffraction pattern will be different to a certain extent. When judging whether the crystal form is the same as any known crystal form, more attention should be paid to the relative positions of peaks rather than relative intensities thereof, which is also well known in the field of crystallography. It should be noted that due to temperature change, sample movement or instrument calibration, the peak position of the characteristic peak can be shifted within an appropriate range, and a measurement error of 2θ value is generally accepted to be ±0.2°.

In addition, DSC can be used to analyze a transition temperature when a resulting crystal absorbs or releases heat due to crystal transformation or crystal melting. For the same crystal form of the same compound, it is well known that the error of crystal transformation temperature and melting point should be limited to 5°C according to crystallography in continuous analysis, and should be limited to 3°C under normal conditions.

Crude product of the single isomer compound shown in formula I used in the embodiments of the present invention was prepared with reference to the method of embodiment 2 disclosed in patent CN 104245707.

### Example 1

The present embodiment provides a preparation method for the crystal form B of Compound I of the present invention as follows:
To a 100mL eggplant flask, 5.0g crude product of the single isomer compound shown in Compound I and 40mL acetone were added, heated to 60°C, stirred until basically dissolved, and filtered while hot to obtain a filtrate which was placed into a 100mL beaker for stirring, slowly cooled to room temperature, and stirred overnight for crystallization. A resulting product was dried to obtain 0.58g solid, with a yield of 58%. The crystal had characteristic peaks at 11.21±0.2° (7.90), 12.61±0.2° (7.02), 14.69±0.2° (6.03), 16.14±0.2° (5.49), 17.81±0.2° (4.98), 19.42±0.2° (4.57), 20.22±0.2° (4.39), 20.76±0.2° (4.27), 22.10±0.2° (4.02), 23.24±0.2° (3.83), 24.31±0.2° (3.66), 27.01±0.2° (3.30), 27.68±0.2° (3.22), 28.57±0.2° (3.12) and 31.09±0.2° (2.88). The DSC spectrogram is shown in FIGURE 2, and there is only one sharp melting endothermic peak at 151.71°C, the TGA spectrogram is shown in FIGURE 3, and the crystal form is defined as crystal form B.

### Example 2

The present embodiment provides a preparation method for the crystal form B of Compound I of the present invention as follows:
To a 10mL eggplant flask, 100mg crude product of the single isomer compound shown in Compound I and 5mL acetone were added, shaken at 60°C for dissolving, and filtered while hot to obtain a filtrate which was placed into a clean 5mL eggplant flask, slowly stood and cooled to room temperature to obtain a single crystal of the compound shown in Compound I with high crystallinity and single crystal diffraction pattern shown in FIGURE 4. After cryogrinding of the single crystal, an X-ray powder diffraction pattern and DSC spectrogram thereof were studied and compared, and a resulting product was determined to be crystal form B.

### Example 3

The present embodiment provides a preparation method for the crystal form B of Compound I of the present invention as follows:
To a 100mL eggplant flask, 1.0g crude product of the single isomer compound shown in Compound I and 35mL acetonitrile were added, heated to 60°C, stirred until basically dissolved, and filtered while hot to obtain a filtrate which was placed into a 100mL beaker for stirring, slowly cooled to room temperature, and stirred overnight for crystallization. A resulting product was dried to obtain 0.42g solid, with a yield of 42%. An X-ray powder diffraction pattern and DSC spectrogram thereof were studied and compared, and a resulting product was determined to be crystal form B.

### Example 4

The present embodiment provides a preparation method for the crystal form B of Compound I of the present invention as follows:
To a 50mL eggplant flask, 1.0g crude product of the single isomer compound shown in Compound I and 15mL ethanol were added, heated to 60°C, stirred until basically dissolved, and filtered while hot to obtain a filtrate which was placed into a 50mL beaker for stirring, slowly cooled to room temperature, and stood at -20°C for crystallization. A resulting product was dried to obtain 0.45g solid, with a yield of 45%. An X-ray powder diffraction pattern and DSC spectrogram thereof were studied and compared, and a resulting product was determined to be crystal form B.

### Example 5

The present embodiment provides a preparation method for the crystal form B of Compound I of the present invention as follows:
To a 50mL eggplant flask, 1.0g crude product of the single isomer compound shown in Compound I and 20mL diethyl ether were added, heated to 30°C, stirred until basically dissolved, and filtered while hot to obtain a filtrate which was placed into a 50mL beaker for stirring, slowly cooled to room temperature, and stirred overnight for crystallization. A resulting product was dried to obtain 0.45g solid, with a yield of 45%. An X-ray powder diffraction pattern and DSC spectrogram thereof were studied and compared, and a resulting product was determined to be crystal form B.

### Example 6

The present embodiment provides a preparation method for the crystal form B of Compound I of the present invention as follows:
To a 50mL eggplant flask, 1.0g crude product of the single isomer compound shown in Compound I and 30mL tetrahydrofuran were added, heated to 60°C, stirred until basically dissolved, and filtered while hot to obtain a filtrate which was placed into a 50mL beaker for stirring, slowly cooled to room temperature, and stirred overnight for crystallization. A resulting product was dried to obtain 0.38g solid, with a yield of 38%. An X-ray powder diffraction pattern and DSC spectrogram thereof were studied and compared, and a resulting product was determined to be crystal form B.

### Example 7

The present embodiment provides a preparation method for the crystal form B of Compound I of the present invention as follows:
To a 50mL eggplant flask, 1.0g crude product of the single isomer compound shown in Compound I and 20mL DMSO were added, heated to 60°C, stirred until basically dissolved, and filtered while hot to obtain a filtrate which was placed into a 50mL beaker for stirring, slowly cooled to room temperature, and stirred in ice bath overnight for crystallization. A resulting product was dried to obtain 0.28g solid, with a yield of 28%. An X-ray powder diffraction pattern and DSC spectrogram thereof were studied and compared, and a resulting product was determined to be crystal form B.

### Comparative example 1

The present embodiment is a comparative example, and provides a preparation method for the crystal form in the prior art (CN 104245707) as follows:
To a 100mL eggplant flask, 1.0g crude product of a racemic mixture (prepared according to the method of example 1 disclosed in CN 104245707) shown in Compound I, 30mL ethyl acetate and 1.5mL methanol were added, heated to 60°C, stirred until basically dissolved, and filtered while hot to obtain a filtrate which was placed into a 100mL beaker for stirring, slowly cooled to room temperature, and stirred overnight for crystallization. A resulting product was dried to obtain 0.32g solid, with a yield of 32%. According to comparison of X-ray powder diffraction patterns and measurement result of melting point (melting range was 135.0-138.0°C), the product was determined as the crystal form described in CN 104245707.

### Comparative example 2

The present embodiment is a comparative example, and provides a preparation method for the crystal form in the prior art (CN 104245707) as follows:
To a 100mL eggplant flask, 1.0g crude product of a racemic mixture (prepared according to the method of example 1 disclosed in CN 104245707) shown in Compound I, 30mL ethyl acetate and 3mL methanol were added, heated to 60°C, stirred until basically dissolved, and filtered while hot to obtain a filtrate which was placed into a 100mL beaker for stirring, slowly cooled to room temperature, and stirred overnight for crystallization. A resulting product was dried to obtain 0.38g solid, with a yield of 38%. According to comparison of X-ray powder diffraction patterns and measurement result of melting point (melting range was 134.5-138.0°C), the product was determined as the crystal form described in CN 104245707.

### Comparative example 3

The present embodiment is a comparative example, and provides a preparation method for the crystal form in the prior art (CN 104245707) as follows:
To a 100mL eggplant flask, 5.0g crude product of a racemic mixture (prepared according to the method of example 1 disclosed in CN 104245707) shown in Compound I, 30mL ethyl acetate and 6mL methanol were added, heated to 60°C, stirred until basically dissolved, and filtered while hot to obtain a filtrate which was placed into a 100mL beaker for stirring, slowly cooled to room temperature, and stirred overnight for crystallization. A resulting product was dried to obtain 0.41g solid, with a yield of 41%. According to comparison of X-ray powder diffraction patterns and measurement result of melting point (melting range was 135.0-138.0°C), the product was determined as the crystal form described in CN 104245707.

### Test 1 Determination of related substances in the crystal forms in the examples

HPLC analysis was performed on the crystal form of the compound shown in Compound I as obtained from each example and each comparative example.

### Instruments and equipment: Agilent-1100 HPLC system;

Chromatographic conditions and system suitability test: Octadecyl silane chemically bonded silica was used as a filler, water (pH value was adjusted to 3.8 with phosphoric acid): acetonitrile (45:55) was used as a mobile phase, and a detection wavelength was 220nm; and number of theoretical plates should not be less than 2000, a resolution should not be less than 2.0, and the resolution with other adjacent impurity peaks should meet the requirements.

Test solution: An appropriate amount of the compound obtained in each example was accurately weighed, dissolved in ethanol absolute and quantitatively diluted to prepare a solution containing about 1mg per 1ml as the test solution.

Reference solution: A total of 1ml reference solution of the compound shown in Compound I was accurately measured, placed into a 100ml volumetric flask, and diluted with ethanol absolute to volume, and shaken up to obtain the reference solution. Reference substance: Calibrated standard substance of Compound I supplied by Chengdu Shibeikang Biomedical Technology Co., Ltd.

Determination method: According to General Chapter 0512 High Performance Liquid Chromatography in Chinese Pharmacopoeia (2015 Edition) Volume IV, 10µl of the test solution and 10µl of the reference solution were accurately measured respectively, injected into the HPLC system separately, and chromatograms were recorded.

**Table 1 Determination results of related substances in the crystal forms in the embodiments**

| No. | Content (%) | Maximum single impurity (%) | Diastereoisomer (%) | Number of impurities |
|---|---|---|---|---|
| Example 1 | 99.82 | <0.10 | <0.30 | 2 |
| Example 2 | 99.92 | <0.10 | <0.30 | 2 |
| Example 3 | 99.94 | <0.10 | <0.30 | 2 |
| Example 4 | 99.89 | <0.10 | <0.30 | 2 |
| Example 5 | 99.87 | <0.10 | <0.30 | 2 |
| Example 6 | 99.91 | <0.10 | <0.30 | 2 |
| Example 7 | 99.88 | <0.10 | <0.30 | 2 |
| Comparative example 1 | 96.89 | >0.50 | >0.45 | 5 |
| Comparative example 2 | 96.43 | >0.50 | >0.45 | 5 |
| Comparative example 3 | 97.73 | >0.50 | >0.45 | 5 |

According to Table 1, purities of the crystal form B prepared by the present invention were greater than 99%, and the maximum single impurity was less than 0.1%, which met the requirements of ICH impurity requirements for active pharmaceutical ingredients (APIs), and diastereoisomer were metabolic components in vivo, and standards thereof could be relatively relaxed according to ICH impurity requirements. Compared with the comparative examples, the crystal form B prepared by the present invention had higher content, lower maximum single impurity and fewer impurities.

According to the ICH impurity requirements of APIs, the crystal form compounds of the comparative examples did not meet the pharmaceutical requirements.

### Test 2 Stability investigation of the crystal forms in the examples

The crystal form B samples obtained from the examples and the comparative examples were placed open and evenly respectively to investigate 5-day stability and 10-day stability of the samples exposed to light (4500Lux) and heated to 40°C and 60°C at high humidity of 75% and 90%, respectively. The testing was performed according to the determination method in Test 1, and HPLC purity test results are shown in the table below.

**Table 2 Determination results of the embodiments when exposed to light (4500Lux)**

| No. | 0 day (%) | 5 days (%) | 10 days (%) |
|---|---|---|---|
| Example 1 | 99.82 | 99.82 | 99.82 |
| Example 2 | 99.92 | 99.92 | 99.92 |
| Example 3 | 99.94 | 99.94 | 99.94 |
| Example 4 | 99.89 | 99.89 | 99.89 |
| Example 5 | 99.87 | 99.87 | 99.86 |
| Example 6 | 99.91 | 99.91 | 99.90 |
| Example 7 | 99.88 | 99.88 | 99.87 |
| Comparative example 1 | 96.89 | 96.80 | 96.75 |
| Comparative example 2 | 96.43 | 96.35 | 96.27 |
| Comparative example 3 | 97.73 | 97.70 | 97.65 |

According to Table 2, the crystal form B prepared by the examples of the present invention and the crystal form compounds of the comparative examples were relatively stable when exposed to light (4500Lux). In comparison, the crystal form B prepared by the examples of the present invention had better stability.

**Table 3 Stability results when heated to 40°C**

| No. | 0 day (%) | 5 days (%) | 10 days (%) |
|---|---|---|---|
| Example 1 | 99.82 | 99.82 | 99.81 |
| Example 2 | 99.92 | 99.92 | 99.91 |
| Example 3 | 99.94 | 99.93 | 99.93 |
| Example 4 | 99.89 | 99.89 | 99.88 |
| Example 5 | 99.87 | 99.87 | 99.87 |
| Example 6 | 99.91 | 99.91 | 99.90 |
| Example 7 | 99.88 | 99.88 | 99.88 |
| Comparative example 1 | 96.89 | 96.31 | 95.35 |
| Comparative example 2 | 96.43 | 96.01 | 94.78 |
| Comparative example 3 | 97.73 | 97.22 | 96.13 |

According to Table 3, the crystal form compounds of the comparative example were relatively sensitive to temperature, and the crystal form B prepared by the examples of the present invention had significantly better stability than the crystal form compounds of the comparative examples.

**Table 4 Stability results when heated to 60°C**

| No. | 0 day (%) | 5 days (%) | 10 days (%) |
|---|---|---|---|
| Example 1 | 99.82 | 99.79 | 99.77 |
| Example 2 | 99.92 | 99.87 | 99.84 |
| Example 3 | 99.94 | 99.90 | 99.88 |
| Example 4 | 99.89 | 99.87 | 99.86 |
| Example 5 | 99.87 | 99.85 | 99.85 |
| Example 6 | 99.91 | 99.89 | 99.88 |
| Example 7 | 99.88 | 99.85 | 99.84 |
| Comparative example 1 | 96.89 | 95.45 | 94.37 |
| Comparative example 2 | 96.43 | 95.23 | 93.89 |
| Comparative example 3 | 97.73 | 96.60 | 95.23 |

According to Table 4, the crystal form compounds of the comparative example were more sensitive to high temperature, and the crystal form B prepared by the examples of the present invention had significantly better stability than the crystal form compounds of the comparative examples.

**Table 5 Stability results at high humidity of 90%**

| No. | 0 day (%) | 5 days (%) | 10 days (%) |
|---|---|---|---|
| Example 1 | 99.82 | 99.82 | 99.81 |
| Example 2 | 99.92 | 99.92 | 99.91 |
| Example 3 | 99.94 | 99.94 | 99.94 |
| Example 4 | 99.89 | 99.89 | 99.89 |
| Example 5 | 99.87 | 99.87 | 99.86 |
| Example 6 | 99.91 | 99.91 | 99.90 |
| Example 7 | 99.88 | 99.88 | 99.87 |
| Comparative example 1 | 96.89 | 96.75 | 96.69 |
| Comparative example 2 | 96.43 | 96.33 | 96.23 |
| Comparative example 3 | 97.73 | 97.66 | 97.58 |

According to Table 5, the crystal form compounds of the comparative example were relatively sensitive to high humidity, and the crystal form B prepared by the examples of the present invention had significantly better stability than the crystal form compounds of the comparative examples.

### Test 3 Determination of melting point data

The crystal form B of Compound I obtained in example 1 was ground and heated to investigate the stability of the crystal form, and melting point data are shown below:

**Table 6 Melting point data of the embodiment**

| Batch No. | Processing method | Melting range |
|---|---|---|
| Example 1 (crystal form B) | Grinding for 10 minutes under nitrogen protection | 148.0-150.5°C |
| | Heating at 80°C for 12 hours under nitrogen protection | 148.2-150.3°C |
| Comparative example(crystal form) | Grinding for 10 minutes under nitrogen protection | 135.0-138.0°C |
| | Heating at 80°C for 12 hours under nitrogen protection | 136.8-139.0°C |

According to Table 6, it is well known that the better the stability of the crystal form, the higher the melting point, and different melting points correspond to different crystal forms. Also according to Table 6, the crystal form B prepared in example 1 of the present invention had a higher melting point than the crystal form compounds of the comparative examples, which further showed that the crystal form B had significantly better stability than the crystal form compounds of the comparative examples.

### Test 4 Study on tabletting performance of the embodiments

Test method:
① After pulverization, raw materials passed through a 80-mesh screen.
② The raw materials and excipients were weighed according to the above formula, the raw materials were mixed with some lactose, and a mixture of the raw materials and lactose was mixed with remaining lactose, microcrystalline cellulose, corn starch and crospovidone evenly, and then mixed with magnesium stearate evenly.
③ Determination of material flowability

Determination method: An iron ring was fixed on an iron stand, a watch glass was placed directly below a funnel, the watch glass was regulated so that an origin thereof was perpendicular to the funnel, multiple batches of materials were fed from the funnel until an edge of the watch glass could not hold the materials, that is, a regular cone was formed, feeding was stopped at that time, a height h of the materials was measured with a ruler, and then an outer diameter R of the watch glass was determined according to equation tangθ = 2h/R. The θ value was calculated to obtain an angle of repose.

**Table 7 Prescription composition of preparation**

| Prescription composition (g/1000 tablets) | Comparative example 1 | Example 1 |
|---|---|---|
| Crystal form of raw materials | Crystal form of comparative example | crystal form B |
| Compound I (calculated as pure Compound I, the same content) | 1 | 1 |
| Lactose | 90 | 90 |
| Microcrystalline cellulose | 20 | 20 |
| Corn starch | 10 | 10 |
| Crospovidone | 8 | 8 |
| Magnesium stearate | 1 | 1 |

**Table 8 Determination results of the angle of repose of total mixed particles**

| | Comparative example 1 | Example 1 |
|---|---|---|
| Angle of repose | 38.3°±0.5 | 30.5°±0.7** |

| | | |
|---|---|---|
| Note: Compared with the comparative example, ** P<0.01. | | |

Conclusion: The test results are shown in Table 8. The mixed material prepared by example 1 had better flowability than the mixed material prepared by the mixture prepared from the crystal forms of the comparative examples, was more conducive to the control of tablet weight variation during tabletting, and was more conducive to automatic and commercial production of tablets. The results showed that the crystal form B of the example 1 had significant flowability advantage (**P<0.01).

### Test 5 Study on pharmacodynamics of the embodiment

Test method: Two tablets of different crystal forms of Compound I were prepared from the excipients of the same type and weight by the same powder tabletting process. One tablet was the tablet of example 1 (the crystal form B), and the other tablet was the tablet of the crystal form of the comparative example. The two tablets were compared in terms of anti-platelet aggregation effect.

Eight male beagles with a weight difference not more than 1kg were randomly divided into two groups according to body weight, with four in each group, and administered in two cycles. In a first cycle, a group of example 1 and a group of the comparative example group were orally given 30mg (calculated as pure compound of formula I, the same content) tablets. Blood was collected from the beagles before and 6 hours after administration to collect platelet-rich plasma. Platelet aggregation was induced with 20µM ADP, and an aggregation rate in the platelet-rich plasma was measured by a platelet aggregation analyzer.

After 14 days of elution, the animals were crossed. Similarly in a second cycle, the group of 14 example 1 and the group of the comparative example were orally given 30mg (calculated as pure compound of formula I, the same content) tablets. Blood was collected from the beagles before and 6 hours after administration to collect platelet-rich plasma. The platelet aggregation was induced with 20µM ADP, and the aggregation rate in the platelet-rich plasma was measured by a platelet aggregation analyzer.

The test results are shown in 9:

**Table 9 Results of pharmacodynamic test of the embodiment (n=8)**

| Group | Route of administration | Dose (single animal) | Platelet aggregation rate before administration (%) | Platelet aggregation rate after administration (%) |
|---|---|---|---|---|
| Tablet of example 1 (crystal form B) | Per os | 30mg | 71.5±13.7 | 4.50±2.12* |
| Tablet of comparative example 1 (crystal form) | Per os | 30mg | 71.0±12.9 | 8.31±4.11 |

| | | | | |
|---|---|---|---|---|
| Note: Compared with the comparative example, ** P<0.05. | | | | |

Conclusion: The test results are shown in Table 9. Compared with the comparative example, there was no significant difference before administration, but 6 hours after administration, the crystal form B of example 1 (crystal form B) had stronger resistance against ADP-induced platelet aggregation (*P<0.05), and had significant advantages. Therefore, the crystal form B is a dominant crystal form with further medicinal potential, and is worthy of further research.

The crystal form B of (S)-2-(2-chlorophenyl)-2-((S)-2-oxo-2,6,7,7a-tetrahydrothiophene[3,2-c]pyridine-5(4H)yl)methyl acetate and the preparation method therefor proposed and disclosed in the present invention can be implemented by those skilled in the art by appropriately changing the raw materials, process parameters and other links with reference to the contents herein.

## Claims

1. A crystal form B of Compound I (a tetrahydrothienopyridine compound), **characterized in that** the crystal form B uses Cu-Kα radiation, and X-ray powder diffraction expressed at a 2θ angle has characteristic peaks at 11.21±0.2°, 12.61±0.2°, 14.69±0.2°, 16.14±0.2°, 17.81 ±0.2°, 20.22±0.2°, and 22.10±0.2°;

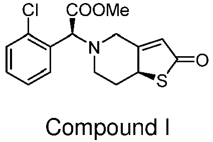

2. The crystal form B according to claim 1, **characterized in that** the crystal form B uses Cu-Kα radiation, and X-ray powder diffraction expressed at the 2θ angle has characteristic peaks at 11.21 ±0.2°, 12.61±0.2°, 14.69±0.2°, 16.14±0.2°, 17.81±0.2°, 20.22±0.2°, 22.10±0.2°, 23.24±0.2°, 27.68±0.2°, and 28.57±0.2°.

3. The crystal form B according to claim 2, **characterized in that** the crystal form B uses Cu-Kα radiation, and X-ray powder diffraction expressed at the 2θ angle has characteristic peaks at 11.21±0.2°, 12.61±0.2°, 14.69±0.2°, 16.14±0.2°, 17.81±0.2°, 19.42±0.2°, 20.22±0.2°, 20.76±0.2°, 22.10±0.2°, 23.24±0.2°, 24.31±0.2°, 27.01±0.2°, 27.68±0.2°, 28.57±0.2°, and 31.09±0.2°.

4. The crystal form B according to claim 1, **characterized in that** X-ray powder diffraction pattern of the crystal form is shown in Figure 1.

5. The crystal form B according to claim 4, **characterized in that** a differential scanning calorimetry spectrogram of the crystal form has an endothermic peak at 151.71±5°C.

6. The crystal form B according to claim 5, **characterized in that** the differential scanning calorimetry spectrogram of the crystal form is shown in Figure 2.

7. The crystal form B according to claim 7, **characterized in that** a thermogravimetric analysis spectrogram of the crystal form is shown in Figure 3.

8. A method for preparing the crystal form B according to any of claims 1-7, **characterized in that** a crude product of any crystal form or amorphous compound of Compound I is dissolved in a solvent in any way, then cooled or concentrated to crystallize, and a resulting product is filtered, washed and dried to obtain the crystal form.

9. A pharmaceutical composition, comprising the crystal form B according to any of claims 1-7 and one or more pharmaceutically acceptable carriers.

10. Use of the crystal form B according to any of claims 1-7 in preparation of drugs for prevention or treatment of cardiac, cerebral and other arterial circulation disorders due to platelet hyperaggregation.

## Patentansprüche

1. Kristallform B der Verbindung I (eine Tetrahydrothienopyridinverbindung), **dadurch gekennzeichnet, dass** die Kristallform B Cu-Ka-Strahlung verwendet und die Röntgenpulverdiffraktion bei einem 2θ-Winkel charakteristische Peaks bei 11,21±0,2°, 12,61±0,2°, 14,69±0,2°, 16,14±0,2°, 17,81±0,2°, 20,22±0,2° und 22,10±0,2° aufweist;

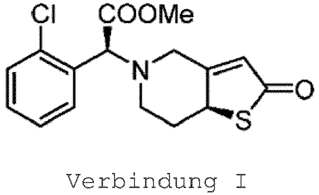

2. Kristallform B nach Anspruch 1, **dadurch gekennzeichnet, dass** für die Kristallform B Cu-Ka-Strahlung verwendet wird und die Röntgenpulverdiffraktion, ausgedrückt im 2θ-Winkel, charakteristische Peaks bei 11,21±0,2°, 12,61±0,2°, 14,69±0,2°, 16,14±0,2°, 17,81±0,2°, 20,22±0,2°, 22,10±0,2°, 23,24±0,2°, 27,68±0,2°, und 28,57±0,2°.

3. Kristallform B nach Anspruch 2, **dadurch gekennzeichnet, dass** für die Kristallform B Cu-Ka-Strahlung verwendet wird und die Röntgenpulverdiffraktion, ausgedrückt im 2θ-Winkel, charakteristische Peaks bei 11,21±0,2°, 12,61±0,2°, 14,69±0,2°, 16,14±0,2°, 17,81±0,2°, 19,42±0,2°, 20,22±0,2°, 20,76±0,2°, 22,10±0,2°, 23,24±0,2°, 24,31±0,2°, 27,01±0,2°, 27,68±0,2°, 28,57±0,2°, und 31,09±0,2°.

4. Kristallform B nach Anspruch 1, **dadurch gekennzeichnet, dass** das Röntgenpulverdiffraktionsmuster der Kristallform in Figur 1 dargestellt ist.

5. Kristallform B nach Anspruch 4, **dadurch gekennzeichnet, dass** ein Differentialscanningkalorimetrie-Spektrogramm der Kristallform einen endothermen Höchstwert bei 151,71±5°C aufweist.

6. Kristallform B nach Anspruch 5, **dadurch gekennzeichnet, dass** das Differentialscanningkalorimetrie-Spektrogramm der Kristallform in Figur 2 dargestellt ist.

7. Kristallform B nach Anspruch 7, **dadurch gekennzeichnet, dass** ein Spektrogramm der thermogravimetrischen Analyse der Kristallform in Figur 3 dargestellt ist.

8. Verfahren zur Herstellung der Kristallform B nach einem der Ansprüche 1-7, **dadurch gekennzeichnet, dass** ein Rohprodukt einer beliebigen Kristallform oder amorphen Verbindung der Verbindung I in einem Lösungsmittel in beliebiger Weise gelöst wird, dann abgekühlt oder konzentriert wird, um zu kristallisieren, und ein resultierendes Produkt filtriert, gewaschen und getrocknet wird, um die Kristallform zu ermitteln.

9. Pharmazeutische Zusammensetzung, umfassend die Kristallform B nach einem der Ansprüche 1-7 und einen oder mehrere pharmazeutisch verträgliche Träger.

10. Verwendung der Kristallform B nach einem der Ansprüche 1-7 zur Herstellung von Medikamenten zur Prävention oder Behandlung von Herz-, Hirn- und anderen arteriellen Durchblutungsstörungen aufgrund von Thrombozytenhyperaggregation.

## Revendications

1. Forme cristalline B du composé I (un composé de tétrahydrothiénopyridine), **caractérisée en ce que** la forme cristalline B utilise un rayonnement Kα du Cu, et une diffraction des rayons X sur poudre exprimée à un angle 2θ présente des pics caractéristiques à 11,21 ± 0,2°, 12,61 ± 0,2°, 14,69 ± 0,2°, 16,14 ± 0,2°, 17,81 ± 0,2°, 20,22 ± 0,2°, et 22,10 ± 0,2° ;

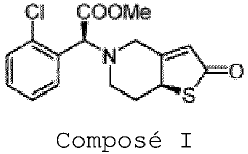

2. Forme cristalline B selon la revendication 1, **caractérisée en ce que** la forme cristalline B utilise un rayonnement Kα du Cu, et une diffraction des rayons X sur poudre exprimée à l'angle 2θ présente des pics caractéristiques à 11,21 ± 0,2°, 12,61 ± 0,2°, 14,69 ± 0,2°, 16,14 ± 0,2°, 17,81 ± 0,2°, 20,22 ± 0,2°, 22,10 ± 0,2°, 23,24 ± 0,2°, 27,68 ± 0,2°, et 28,57 ± 0,2°.

3. Forme cristalline B selon la revendication 2, **caractérisée en ce que** la forme cristalline B utilise un rayonnement Kα du Cu, et une diffraction des rayons X sur poudre exprimée à l'angle 2θ présente des pics caractéristiques à 11,21 ± 0,2°, 12,61 ± 0,2°, 14,69 ± 0,2°, 16,14 ± 0,2°, 17,81 ± 0,2°, 19,42 ± 0,2°, 20,22 ± 0,2°, 20,76 ± 0,2°, 22,10 ± 0,2°, 23,24 ± 0,2°, 24,31 ± 0,2°, 27,01 ± 0,2°, 27,68 ± 0,2°, 28,57 ± 0,2°, et 31,09 ± 0,2°.

4. Forme cristalline B selon la revendication 1, **caractérisée en ce qu'**un diagramme de diffraction des rayons X sur poudre de la forme cristalline est représenté sur la figure 1.

5. Forme cristalline B selon la revendication 4, **caractérisée en ce qu'**un spectrogramme de calorimétrie différentielle à balayage de la forme cristalline présente un pic endothermique à 151,71 ± 5 °C.

6. Forme cristalline B selon la revendication 5, **caractérisée en ce que** le spectrogramme de calorimétrie différentielle à balayage de la forme cristalline est représenté sur la figure 2.

7. Forme cristalline B selon la revendication 7, **caractérisée en ce qu'**un spectrogramme d'analyse thermogravimétrique de la forme cristalline est représenté sur la figure 3.

8. Procédé de préparation de la forme cristalline B selon l'une quelconque des revendications 1 à 7, **caractérisé en ce qu'**un produit brut de n'importe quelle forme cristalline ou d'un composé amorphe du composé I est dissous dans un solvant de quelque manière que ce soit, puis refroidi ou concentré pour cristalliser, et un produit résultant est filtré, lavé et séché pour obtenir la forme cristalline.

9. Composition pharmaceutique, comprenant la forme cristalline B selon l'une quelconque des revendications 1 à 7 et un ou plusieurs supports pharmaceutiquement acceptables.

10. Utilisation de la forme cristalline B selon l'une quelconque des revendications 1 à 7 dans la préparation de médicaments pour la prévention ou le traitement des troubles de la circulation cardiaque, des troubles de la circulation cérébrale et d'autres troubles de la circulation artérielle dus à une hyperagrégation plaquettaire.
